# EUROPÄISCHE PATENTSCHRIFT

(11) **EP 0 893 434 B1**
(45) Veröffentlichungstag und Bekanntmachung des Hinweises auf die Patenterteilung: **26.02.2003**
(21) Anmeldenummer: 98115073.3
(22) Anmeldetag: 03.06.1991
(51) Int. Cl.: C07C 251/60

(54) **Zwischenprodukte für die Herstellung von Oximäthern**
Intermediates for the preparation of oximethers
Produits intermédiaires pour la préparation d'oximethers

(30) Priorität: 05.06.1990 CH 189190; 23.04.1991 CH 120891
(43) Veröffentlichungstag der Anmeldung: 27.01.1999
(62) Teilanmeldung aus: 95112791.9
(73) Patentinhaber: Bayer CropScience AG, 40789 Monheim (DE)
(72) Erfinder: Isenring, Hans Peter, Dr., 4450 Sissach (CH); Drechsel-Weiss, Bettina, 3800 Unterseen (CH)
(74) Vertreter: Zumstein, Fritz, Dr.

(56) Entgegenhaltungen:
- EP-A- 0 363 818

## Beschreibung

Die vorliegende Erfindung betrifft Verbindungen der allgemeinen Formel IIIc 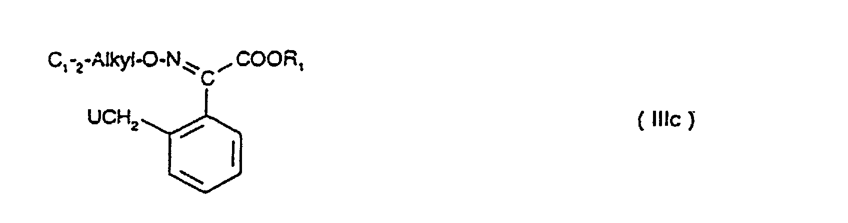 worin
- R₁: für C₁₋₄-AIkyl und
- U: für Chlor, Brom, Jod, Mesyloxy, Benzolsulfonyloxy oder Tosyloxy steben mit der Massgabe, dass R₁ und C₁₋₂-Alkyl nicht gleichzeitig Methyl bedeuten wenn U für Brom steht.

Die erfindungsgemäßen Verbindungen sind brauchbar als Ausgangsprodukte für die Herstellung von fungizid wirksamen.

Oximäthern der allgemeinen Formel 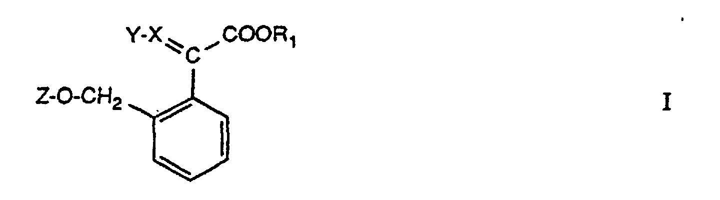 worin
- R₁: C₁₋₄-Alkyl bedeutet,
- (Y-X): C₁₋₂-alkyl-ON= bedeutet
- und Z: für eine Aldimino- oder Ketiminogruppe steht, und zwar insbesondere für eine Gruppe 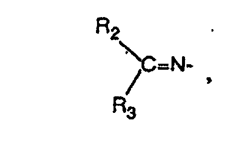
- worin R₂: Wasserstoff, C₁₋₄-Alkyl, C₁₋₄-Halogenalkyl, C₃₋₆-Cycloalkyl, C₂₋₄-Alkenyl, C₂₋₄-Alkinyl, C₁₋₂-Alkoxymethyl, C₁₋₂-Alkylthiomethyl, C₁₋₄-Alkylsulfonyl, C₁₋₃-Alkoxy, C₁₋₃-Alkylthio oder Cyano
- und R₃: C₁₋₆-Alkyl, Aryl-C₁₋₄-alkyl, Heteroaryl-C₁₋₄-alkyl, C₂₋₁₂-Alkenyl,Aryl-C₂₋₄-alkenyl, Aryloxy-C₁₋₄-alkyl, Heteroaryloxy-C₁₋₄-alkyl, Heteroaryl-C₂₋₄-alkenyl, C₃₋₆-Cycloalkyl, Aryl, Heteroaryl, C₂₋₅-Alkanoyl, Aroyl oder Heteroaroyl bedeuten,
- oder R₂: und R₃ zusammen mit dem Kohlenstoffatom, an das sie gebunden sind, einen gegebenenfalls substituierten, gegebenenfalls ein Sauerstoffatom, Schwefelatom und/oder Stickstoffatom enthaltenden vier- bis siebengliedrigen gesättigten oder ungesättigten Ring bilden, der zudem einen gegebenenfalls substituierten ankondensierten Benzolring aufweisen kann.

Die Verbindungen der allgemeinen Formel I besitzen fungizide Eigenschaften und eignen sich als fungizide Wirkstoffe, insbesondere zur Verwendung in der Landwirtschaft und im Gartenbau.

Aufgrund der erfindungsgemäßen Verbindungen der Formel IIIc werden ermöglicht: ein Verfahren zur Herstellung der Verbindungen der allgemeinen Formel I, fungizide Mittel, die solche Verbindungen als Wirkstoffe enthalten, sowie die Verwendung solcher Verbindungen und Mittel zur Bekämpfung von Fungi in der Landwirtschaft und im Gartenbau.

Bei den so herstellbaren Verbindungen der Formel I handelt es sich insbesondere um Oximäther der Formel I
worin
- R₁: C₁₋₄-Alkyl bedeutet,
- (Y-X): C₁₋₂-alkyl-ON= bedeutet
- und Z: für eine Aldimino- oder Ketiminogruppe steht, und zwar insbesondere für eine Gruppe 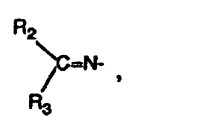
- worin R₂: Wasserstoff, C₁₋₄-Alkyl, C₁₋₄-Halogenalkyl, C₃₋₆-Cycloalkyl
- und R₃: C₁₋₆-Alkyl, Aryl-C₁₋₄-alkyl, Heteroaryl-C₁₋₄-alkyl, C₂₋₆-Alkenyl,Aryl-C₂₋₄-alkenyl, Heteroaryl-C₂₋₄-alkenyl, C₃₋₆-Cycloalkyl, Aryl, Heteroaryl, C₂₋₅-Alkanoyl, Aroyl oder Heteroaroyl bedeuten,
- oder R₂: und R₃ zusammen mit dem Kohlenstoffatom, an das sie gebunden sind, einen gegebenenfalls substituierten, gegebenenfalls ein Sauerstoff- oder Schwefelatom enthaltenden vier- bis siebengliedrigen gesättigten Ring bilden, der zudem einen gegebenenfalls substituierten ankondensierten Benzolring aufweisen kann.

In der obigen Formel I und im folgenden können sämtliche Gruppen "Alkyl" und "Alkenyl", als solche oder als Teil grösserer Gruppen, z.B. Heteroarylalkyl, je nach Anzahl der Kohlenstoffatome geradkettig oder verzweigt sein. Zudem können die Alkenylgruppen eine oder mehrere Doppelbindungen aufweisen. Halogen als Substituent bedeutet Fluor, Chlor, Brom oder Jod, wobei Fluor, Chlor und Brom bevorzugt sind. Eine Halogen-alkylgruppe kann einen oder mehrere gleiche oder verschiedene Halogensubstituenten aufweisen. Unter Aryl ist insbesondere Phenyl, Naphthyl, Phenanthryl oder Fluorenyl zu verstehen. Heteroaryl bedeutet eine heterocyclische Gruppe mit aromatischem Charakter und 1-3 Heteroatomen N, O und /oder S. Bevorzugt sind Triazol oder andere Fünfringe und Sechsringe mit 1-2 Heteroatomen, die ihrerseits zusätzlich einen oder zwei ankondensierte Benzolringe besitzen können.

Als Beispiele, die keine limitierende Bedeutung besitzen, die aber vereinfachend im folgenden als "Gruppe Het*" bezeichnet werden sollen, seien Pyrrolyl, Pyridyl, Furyl, Thienyl, Isoxazolyl, Thiazolyl, Pyrazinyl, Pyridazinyl, Imidazolyl, Pyrimidinyl oder TriazolyI, oder eine solche Gruppe mit ankondensiertem Benzol, z.B. Chinolinyl, Chinoxalinyl, Benzofuryl, Benzothienyl oder Dibenzofuryl genannt. Sinngemäss gilt dies auch für "Aryl" oder "Heteroaryl" als Teil einer grösseren Gruppe, z.B. Aralkyl bzw. Heteroarylalkyl. Die Aryl- und Heteroarylgruppen können jeweils einen oder mehrere der folgenden Substituenten aufweisen:

Halogen, C₁₋₄-Alkyl, C₁₋₄-Halogenalkyl, Aryl-C₁₋₄-alkyl, Aryloxy-C₁₋₄-alkyl, C₂₋₄-Alkenyl, Aryl-C₂₋₄-alkenyl, C₂₋₄-Alkinyl, C₃₋₆-Cycloalkyl, Aryl, C₁₋₄-Alkoxy, C₁₋₄-Halogen-alkoxy, Aryl-C₁₋₄-alkoxy, C₁₋₄-Alkylthio, Aryloxy, Cyano, Nitro,C₂₋₄-Halogenalkenyl, C₂₋₄-Halogenalkinyl, C₂₋₄-Alkenyloxy, C₂₋₄-Halogenalkenyloxy, C₃₋₄-Alkinyloxy, C₃₋₄-Halogenalkinyloxy, Cyclopropylmethoxy, Cyclopropyl (gegebenenfalls ein- bis dreifach substituiert durch Halogen und/oder Methyl), Cyanomethoxy (-OCH₂CN), C₁₋₄-Alkoxymethyl, C₁₋₄-Alkylthiomethyl, C₁₋₄-Alkylsulfinylmethyl, C₁₋₄-Alkylsulfonylmethyl, Arylthio, Thiocyanato, C₁₋₄-Alkoxyiminomethyl, C₁₋₄-Alkanoyloxy, C₁₋₄-Alkoxycarbonyl;
sowie auch einen Heteroarylrest, einen Heteroaryl-C₁₋₄-alkylrest, einen Heteroaryloxy-C₁₋₄-alkylrest, einen Heteroaryl-C₂₋₄-aIkenylrest, einen Heteroaryl-C₁₋₄-alkoxyrest oder einen Heteroaryloxyrest; wobei hierin unter dem Begriff Heteroaryl ein Vertreter der obengenannten "Gruppe Het*" zu verstehen ist.

Fast alle der für Aryl- und Heteroarylgruppen vorgenannten Substituenten können ein- bis zweimal auftreten, bevorzugt einmal, mit Ausnahme von C₁₋₄-Alkyl, das bis zu vierfach als Substituent in Frage kommt, und Halogen, das bis zu dreifach, im Falle von Fluor auch bis zu fünffach vorkommen kann.

Der bevorzugte Arylrest ist Phenyl, gleichgültig, ob er allein oder als Teil eines anderen Substituenten in Erscheinung tritt. Aroyl ist demgemäss bevorzugt Benzoyl.

C₂-Alkanoyl bedeutet AcetyL Unter Halogenalkyl sind Alkylgruppen zu verstehen, die bis zu sechsfach gleich oder verschieden durch F, Cl, Br und/oder J substituiert sind. Beispiele von Halogenalkylgruppen allein oder als Teil eines anderen Substituenten (wie Halogenalkoxy) sind CH₂Cl, CHCl₂, CCl₃, CHBr₂, CH₂CH₂Cl, CHCl-CHCl₂, CF₂Cl, CH₂J, CF₃, C₂F₅, CF₂-CF₂Cl, CHF₂, CH₂F, CF₂CHFCF₃.

Bevorzugt sind Trifluormethyl, Difluormethoxy und Trifluormethoxy.

Zudem können die Arylgruppen (insbesondere Phenyl) einen ein oder zwei Sauerstoffatome aufweisenden fünf-, sechs- oder siebengliedrigen gesättigten oder ungesättigten Ring tragen, der gegebenenfalls ein- oder mehrfach mit Methyl, Methoxy, Phenyl, Halogen, Cyano oder Oxo (C=O) substituiert sein kann. Beispiele solcher Gruppen sind 5-Benzofuryl, 6-Benzodioxanyl und 5-(1,3-Benzodioxolyl).

Im Falle, dass R₂ und R₃ zusammen mit dem Kohlenstoffatom, an das sie gebunden sind, einen gegebenenfalls substituierten Ring bilden, wie dieser oben näher beschrieben ist, kommen als Substituenten des Ringes 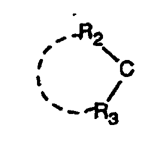 insbesondere C₁₋₆-Alkyl oder gegebenenfalls substituiertes Phenyl in Frage. Auch der allfällig vorhandene ankondensierte Benzolring kann substituiert sein. Als Substituenten der Phenylgruppe bzw. des Benzolrings selber kommen die oben im Zusammenhang mit der Arylgruppe genannten in Betracht.

Falls in den Verbindungen der Formel I asymmetrische Kohlenstoffatome vorliegen, treten die Verbindungen in optisch aktiver Form auf. Allein aufgrund des Vorhandenseins der aliphatischen bzw. Imino-Doppelbindung X=C und der Imino-Doppelbindung der Aldimino- oder Ketiminogruppe Z treten die Verbindungen auf jeden Fall in der [E]- oder [Z]-Form auf. Ferner kann Atropisomerie auftreten. Die Formel I soll all diese möglichen isomeren Formen sowie deren Gemische, z.B. racemische Gemische und beliebige [E/Z]-Gemische, umfassen.

Bei den Verbindungen der Formel I bedeutet R₁ vorzugsweise Methyl. Unabhängig davon bedeutet (Y-X) vorzugsweise Methoxyimino (N-OCH₃).

In der Gruppe (R₂)(R₃)C=N- ist R₂ vorzugsweise Wasserstoff, C₁₋₄-Alkyl (insbesondere Methyl oder Äthyl), C₁₋₄-Halogenalkyl (insbesondere Trifluoromethyl) oder C₃₋₆-Cycloalkyl (insbesondere Cyclopropyl) und R₃ ist vorzugsweise gegebenenfalls substituiertes Phenyl, Naphthyl (insbesondere β-Naphthyl) oder Benzyl, wobei allfällige Substituenten vorzugsweise bis drei gleiche oder verschiedene Halogenatome (insbesondere Fluor, Chlor und/oder Brom), C₁₋₄-Alkylgruppen (insbesondere Methyl), C₁₋₄-Halogenalkylgruppen (insbesondere Trifluormethyl), C₁₋₄-Halogenalkoxygruppen (insbesondere Trifluormethoxy) und Alkylendioxy (insbesondere 3,4-Methylendioxy) sind, oder Heteroaryl, insbesondere gegebenenfalls mit bis zwei Methylgruppen substituiertes Furyl, gegebenenfalls mit Chlor oder Methyl substituiertes Thienyl, Pyridyl oder Benzofuryl.

Falls R₃ Heteroaryl bedeutet, ist R₂ vorzugsweise Methyl.

Weitere Vertreter von Verbindungen der Formel I sind:
diejenigen Verbindungen der Formel I, in denen R₁ Methyl, (Y-X) Methoxyimino (=N-OCH₃), Z eine Gruppe (R₂)(R₃)C=N-, R₂ Methyl und R₃ 4-Chlor-3-trifluormethylbenzyl, Phenyl, 3-Chlorphenyl, 3,5-Dichlorphenyl, 2-Fluor-5-methylphenyl, 3-Trifluormethoxy-phenyl oder 5-Chlor-2-thienyl bedeuten;
diejenigen Verbindungen der Formel I, in denen R₁ Methyl, (Y-X) Methoxyimino, Z eine Gruppe (R₂)(R₃)C=N-, R₂ Trifluormethyl und R₃ 2-(β-Naphthyl)-äthenyl, Phenyl, 3-Chlorphenyl, 4-Chlorphenyl, p-Tolyl, α,α,α-Trifluor-m-tolyl, β-Naphthyl oder 2-Pyridyl bedeuten;
diejenigen Verbindungen der Formel I, in denen R₁ Methyl, (Y-X) Methoxyimino, Z eine Gruppe R₂R₃C=N-, R₂ Cyclopropyl und R₃ 3-Chlorphenyl, 4-Chlorphenyl, 3-Bromphenyl, α,α,α-Trifluor-m-tolyl, 4-Phenoxyphenyl oder β-Naphthyl bedeuten;

Das genannte Verfahren zur Herstellung der Verbindungen der Formel I ist dadurch gekennzeichnet, dass man ein Oxim Z-OH, insbesondere ein Oxim der allgemeinen Formel 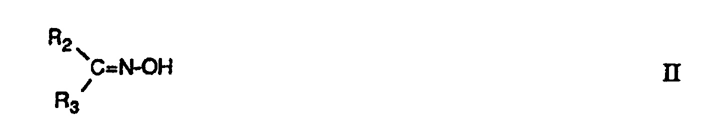 worin R₂ und R₃ die oben angegebenen Bedeutungen besitzen,
mit einem Benzylalkoholderivat der allgemeinen Formel 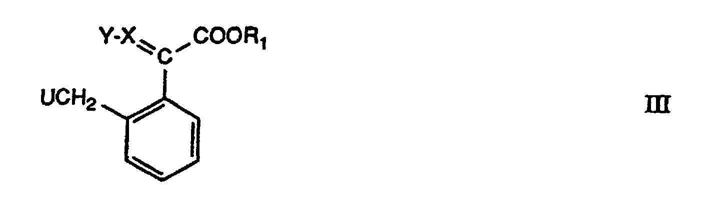 worin R₁ und Y-X die oben, angegebenen Bedeutungen besitzen und U eine Abgangsgruppe bedeutet, zur Reaktion bringt.

Bei dieser Reaktion handelt es sich um eine nucleophile Substitution, die unter den diesbezüglich üblichen Reaktionsbedingungen durchgeführt werden kann. Unter der im Benzylalkoholderivat der Formel III vorhandenen Abgangsgruppe U ist vorzugsweise Chlor, Brom, Jod, Mesyloxy, Benzolsulfonyloxy oder Tosyloxy zu verstehen. Die Umsetzung erfolgt zweckmässigerweise in einem inerten organischen Verdünnungsmittel, wie einem cyclischen Äther, z.B. Tetrahydrofuran oder Dioxan; Aceton, Dimethylformamid oder Dimethylsulfoxid in Gegenwart einer Base, wie Natriumhydrid, Natrium- oder Kaliumcarbonat, eines tertiären Amins, z.B. eines Trialkylamins, insbesondere Diazabicyclononan oder Diazabicycloundecan, oder Silberoxid, bei Temperaturen zwischen -20°C und 80°C, vorzugsweise im Temperaturbereich von 0°C bis 20°C.

Als Alternative kann die Umsetzung unter Phasentransferkatalyse in einem organischen Lösungsmittel, wie beispielsweise Methylenchlorid, in Gegenwart einer wässrigen basischen Lösung, z.B. Natriumhydroxidlösung, sowie eines Phasentransferkatalysators, wie beispielsweise Tetrabutylammoniumhydrogensulfat, bei Raumtemperatur erfolgen [siehe beispielsweise W.E. Keller, "Phasen-Transfer Reactions", Fluka-Compendium Vol. I und II, Georg Thieme Verlag, Stuttgart (1986/1987), in dem insbesondere Chemistry Letters 1980, Seiten 869-870, erwähnt ist].

Die Isolierung und Reinigung der so hergestellten Verbindungen der Formel I kann nach an sich bekannten Methoden erfolgen. Ebenfalls nach an sich bekannten Methoden können allfällig erhaltene Isomerengemische, z.B. E/Z-Isomerengemische, in die reinen Isomeren aufgetrennt werden, beispielsweise durch Chromatographie oder fraktionierte Kristallisation.

Die als Ausgangsmaterialien im genannten Verfähren verwendeten Oxime Z-OH, z.B. der Formel II, sind entweder bekannt oder können nach an sich bekannten Methoden hergestellt werden, beispielsweise durch Umsetzung der entsprechenden Carbonylverbindung R₂R₃C=O mit Hydroxylaminhydrochlorid in Gegenwart einer Base, z.B. Natrium- oder Kaliumhydroxid oder Pyridin. Weitere Methoden finden sich in Houben-Weyl, "Methoden der Organischen Chemie", Band X/4, Seiten 3-308 (1968) ("Herstellung und Umwandlung von Oximen").

Weitere Ausgangsmaterialien der Formel III sind die 2-(2-UCH₂-phenyl)-glyoxyIsäure-alkylester -O-(C₁₋₂-alkyl)oxime der Formel IIIc

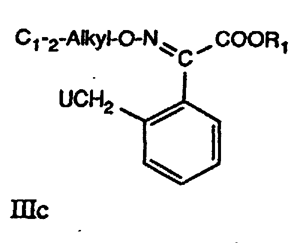

Diese Verbindungen der Formel IIIc sind Gegenstand der vorliegenden Erfindung und können nach an sich bekannten Methoden hergestellt werden. Ferner ist in der europäischen Patentpublikation EP 363,818 und in Angew. Chem. 71, 349-365 (1959) die Herstellung von 2-(2-Brommethyl-phenyl)-glyoxylsäure-methylester-O-methyloxim beschrieben. Nur die neuen Verbindungen der Formel IIIc (vergleiche Patentanspruch) sind Gegenstand der vorliegenden Erfindung.

Die genannten Verbindungen der allgemeinen Formel I besitzen fungizide Wirkung und können dementsprechend zur Bekämpfung bzw. Verhütung von Pilzbefall in der Landwirtschaft, im Gartenbau sowie im Holzschutz Verwendung finden. Sie eignen sich insbesondere zur Hemmung des Wachstums oder zur Vernichtung von phytopathogenen Pilzen auf Pflanzenteilen, z.B. Blättern, Stengeln, Wurzeln, Knollen, Früchten oder Blüten, und auf Saatgut sowie von im Erdboden auftretenden Schadpilzen. Ferner können mit den erfindungsgemässen Verbindungen holzabbauende und holzverfärbende Pilze bekämpft werden. Die genannten Verbindungen der allgemeinen Formel I sind beispielsweise wirksam bei der Bekämpfung von Pilzen der Klassen Deuteromycetes, Ascomycetes, Basidiomycetes und Phycomycetes.

Besonders eignen sich die genannten Verbindungen der allgemeinen Formel I zur Bekämpfung der folgenden Schaderreger:
Echte Mehltaupilze (z.B. Erysiphe graminis, Erysiphe cichoracearum, Podosphaera leucotricha, Uncinula necator, Sphaerotheca spp.)
Rostpilze (z.B. Puccinia tritici, Puccinia recondita, Puccinia hordei, Puccinia coronata, Puccinia striiformis, Puccinia arachidis, Hemileia vastatrix, Uromyces fabae)
Schorfpilze (z.B. Venturia inaequalis)
Cercospora spp. (z.B. Cercospora arachidicola, Cercospora beticola)
Mycosphaerella spp. (z.B. Mycosphaerella fijiensis)
Alternaria spp. (z.B. Alternaria brassicae, Alternaria mali)
Septoria spp. (z.B. Septoria nodorum)
Heminthosporium spp. (z.B. Helminthosporium teres, Helminthosporium oryzea)
Plasmopara spp. (z.B. Plasmopara viticola)
Pseudoperonospora spp. (z.B. Pseudoperonospora cubensis)
Phytophthora spp. (z.B. Phytophthora infestans)
Pseudocercosperella spp. (z.B. Pseudocercosperella herpotrichoides)
Piricularia spp. (z.B. Piricularia oryzae)

Ferner wirken die Verbindungen beispielsweise gegen Pilze der Gattungen Tilletia, Ustilago, Rhizoctonia, Verticillium, Fusarium, Pythium, Gaeumannomyces, Sclerotinia, Monilia, Botrytis, Peronospora, Bremia, Gloeosporium, Cercosporidium, Penicillium, Ceratocystis, Rhynchosporium, Pyrenophora, Diaporthe, Ramularia und Leptosphaeria. Gewisse Vertreter der erfindungsgemässen Verbindungen besitzen zudem Wirkung gegen holzschädigende Pilze, wie beispielsweise der Gattungen Coniophora, Gloeophyllum, Poria, Merulius, Trametes, Aureobasidium, Sclerophoma und Trichoderma.

Die genannten Verbindungen der Formel I zeichnen sich durch prophylaktische und kurative, vor allem aber durch deutliche systemische Wirkung aus.

Sie wirken gegen phytopathogene Pilze unter Gewächshausbedingungen bereits bei Konzentrationen von 0,5 mg bis 500 mg Wirkstoff pro Liter Spritzbrühe. Im Freiland werden vorteilhaft Dosierungen von 20 g bis 1 kg Wirkstoff der Formel I pro Hektar und Behandlung zur Anwendung gebracht. Zur Bekämpfung von samen- oder bodenbürtigen Pilzen im Beizverfahren werden mit Vorteil Dosierungen von 0,001 g bis 1,0 g Wirkstoff der Formel I pro kg Samen verwendet.

Die genannten Verbindungen der allgemeinen Formel I können zu verschiedenartigen Mitteln, z.B. Lösungen, Suspensionen, Emulsionen, emulgierbaren Konzentraten und pulverförmigen Präparaten, formuliert werden. Die genannten fungiziden Mittel sind dadurch gekennzeichnet, dass sie eine wirksame Menge mindestens einer Verbindung der allgemeinen Formel I, wie oben definiert, sowie Formulierungshilfsstoffe enthalten. Die Mittel enthalten zweckmässigerweise zumindest einen der folgenden Formulierungshilfsstoffe:

Feste Trägerstoffe; Lösungs- bzw. Dispersionsmittel; Tenside (Netzmittel und Emulgatoren); Dispergiermittel (ohne Tensidwirkung); und Stabilisatoren.

Als feste Trägerstoffe kommen im wesentlichen in Frage: natürliche Mineralstoffe, wie Kaolin, Tonerden, Kieselgur, Talkum, Bentonit, Kreide, z.B. Schlämmkreide, Magnesiumcarbonat, Kalkstein, Quarz, Dolomit, Attapulgit, Montmorillonit und Diatomeenerde; synthetische Mineralstoffe, wie hochdisperse Kieselsäure, Aluminiumoxid und Silikate; organische Stoffe, wie Cellulose, Stärke, Harnstoff und Kunstharze; und Düngemittel, wie Phosphate und Nitrate, wobei solche Trägerstoffe z.B. als Granulate oder Pulver vorliegen können.

Als Lösungs- bzw. Dispersionsmittel kommen im wesentlichen in Frage: Aromaten, wie Toluol, Xylole, Benzol und Alkylnaphthaline; chlorierte Aromaten und chlorierte aliphatische Kohlenwasserstoffe, wie Chlorbenzole, Chlorethylene und Methylenchlorid; aliphatische Kohlenwasserstoffe, wie Cyclohexan und Paraffine, z.B. Erdölfraktionen; Alkohole, wie Butanol und Glykol, sowie deren Ether und Ester; Ketone, wie Aceton, Methylethylketon, Methylisobutylketon und Cyclohexanon; und stark polare Lösungs- bzw. Dispersionsmittel, wie Dimethylformamid, N-Methylpyrrolidon und Dimethylsulfoxid, wobei solche Lösungs- bzw. Dispersionsmittel vorzugsweise Flammpunkte von mindestens 30°C und Siedepunkte von mindestens 50°C aufweisen, und Wasser. Unter den Lösungs- bzw. Dispersionsmitteln kommen auch in Frage sogenannte verflüssigte gasförmige Streckmittel oder Trägerstoffe, die solche Produkte sind, welche bei Raumtemperatur und unter Normaldruck gasförmig sind. Beispiele solcher Produkte sind insbesondere Aerosol-Treibgase, wie (Halogen)Kohlenwasserstoffe. Im Falle der Benutzung von Wasser als Lösungsmittel können z.B. auch organische Lösungsmittel als Hilfslösungsmittel verwendet werden.

Die Tenside (Netzmittel und Emulgatoren) können nicht-ionische Verbindungen sein, wie Kondensationsprodukte von Fettsäuren, Fettalkoholen oder fettsubstituierten Phenolen mit Ethylenoxid; Fettsäureester und -ether von Zuckern oder mehrwertigen Alkoholen; die Produkte, die aus Zuckern oder mehrwertigen Alkoholen durch Kondensation mit Ethylenoxid erhalten werden; Blockcopolymere von Ethylenoxid und Propylenoxid; oder Alkyldimethylaminoxide.

Die Tenside können auch anionische Verbindungen darstellen, wie Seifen; Fettsulfatester, z.B. Dodecylnatriumsulfat, Octadecylnatriumsulfat und Cetylnatriumsulfat; Alkylsulfonate, Arylsulfonate und fettaromatische Sulfonate, wie Alkylbenzolsulfonate, z.B. Calcium-dodecylbenzolsulfonat, und Butylnaphthalinsulfonate; und komplexere Fettsulfonate, z.B. die Amidkondensationsprodukte von Ölsäure und N-Methyltaurin und das Natriumsulfonat von Dioctylsuccinat.

Die Tenside können schliesslich kanonische Verbindungen sein, wie Alkyldimethylbenzylammoniumchloride, Dialkyldimethylammoniumchloride, AIkyItrimethylammoniumchloride und äthoxylierte quaternäre Ammoniumchloride.

Als Dispergiermittel (ohne Tensidwirkung) kommen im wesentlichen in Frage: Lignin, Natrium- und Ammoniumsalze von Ligninsulfonsäure, Natriumsalze von Maleinsäureanhydrid-Diisobutylen-Copolymeren, Natrium- und Ammoniumsalze von sulfonierten Polykondensationsprodukten aus Naphthalin und Formaldehyd, und Sulfitablaugen.

Als Dispergiermittel, die sich insbesondere als Verdickungs- bzw. Antiabsetzmittel eignen, können z.B. Methylcellulose, Carboxymethylcellulose, Hydroxyethylcellulose, Polyvinylalkohol, Alginate, Caseinate und Blutalbumin eingesetzt werden.

Beispiele von geeigneten Stabilisatoren sind säurebildende Mittel, z.B. Epichlorhydrin, Phenylglycidether und Soyaepoxide; Antioxidantien, z.B. Gallussäureester und Butylhydroxytoluol; UV-Absorber, z.B. substituierte Benzophenone, Diphenylacrylnitrilsäureester und Zimtsäueeester; und Deaktivatoren, z.B. Salze der Ethylendiamintetraessigsäure und Polyglykole.

Die genannten fungiziden Mittel können neben den Wirkstoffen der Formel I auch andere Wirkstoffe enthalten, z.B. anderweitige fungizide Mittel, insektizide und akarizide Mittel, Bakterizide, Pflanzenswachstumsregulatoren und Düngemittel. Solche Kombinationsmittel eignen sich zur Verbreiterung des Wirkungsspektrums oder zur spezifischen Beeinflussung des Pflanzenwachstums.

Im allgemeinen enthalten die genannten Mittel, je nach deren Art, zwischen 0,0001 und 85 Gewichtsprozent an genannter Verbindung bzw. genannten Verbindungen der allgemeinen Formel I als Wirkstoff(en). Sie können in einer Form vorliegen, die sich für die Lagerung und den Transport eignet. In solchen Formen, z.B. emulgierbaren Konzentraten, ist die Wirkstoffkonzentration normalerweise im höheren Bereich des obigen Konzentrationsintervalls. Diese Formen können dann mit gleichen oder verschiedenen Formulierungshilfsstoffen bis zu Wirkstoffkonzentrationen verdünnt werden, die sich für den praktischen Gebrauch eignen, und solche Konzentrationen liegen normalerweise im niedrigeren Berich des obigen Konzentrationsintervalls. Emulgierbare Konzentrate enthalten im allgemeinen 5 bis 85 Gewichtsprozent, vorzugsweise 25 bis 75 Gewichtsprozent, der genannten Verbindung(en) der allgemeinen Formel I. Als Anwendungsformen kommen u.a. gebrauchsfertige Lösungen, Emulsionen und Suspensionen, die sich beispielsweise als Spritzbrühen eignen, in Frage. In solchen Spritzbrühen können. z.B. Konzentrationen zwischen 0,0001 und 20 Gewichtsprozent vorliegen. Im Ultra-Low-Volume-Verfahren können Spritzbrühen formuliert werden, in denen die Wirkstoffkonzentration vorzugsweise von 0,5 bis 20 Gewichtsprozent beträgt, während die im Low-Volume-Verfahren und im High-Volume-Verfahren formulierten Spritzbrühen vorzugsweise eine Wirkstoffkonzentration von 0,02 bis 1,0 bzw. 0,002 bis 0,1 Gewichtsprozent aufweisen.

Die genannten fungiziden Mittel können dadurch hergestellt werden, dass man mindestens eine genannte Verbindung der allgemeinen Formel I mit Formulierungshilfsstoffen vermischt.

Die Herstellung der Mittel kann in bekannter Weise durchgeführt werden, z.B. durch Vermischen der Wirkstoffe mit festen Trägerstoffen, durch Auflösen oder Suspendieren in geeigneten Lösungs- bzw. Dispersionsmitteln, eventuell unter Verwendung von Tensiden als Netzmitteln oder Emulgatoren oder von Dispergiermitteln, durch Verdünnen bereits vorbereiteter emulgierbarer Konzentrate mit Lösungs- bzw. Dispersionsmitteln, usw.

Im Falle von pulverförmigen Mitteln kann der Wirkstoff mit einem festen Trägerstoff vermischt werden, z.B. durch Zusammenmahlen; oder man kann den festen Trägerstoff mit einer Lösung oder Suspension des Wirkstoffs imprägnieren und dann das Lösungs- bzw. Dispersionsmittel durch Abdunsten, Erhitzen oder durch Absaugen unter vermindertem Druck entfernen. Durch Zusatz von Tensiden bzw. Dispergiermitteln kann man solche pulverförmige Mittel mit Wasser leicht benetzbar machen, so dass sie in wässrige Suspensionen, die sich z.B. als Spritzmittel eignen, übergeführt werden können.

Die genannten Verbindungen der allgemeinen Formel I können auch mit einem Tensid und einem festen Trägerstoff zur Bildung eines netzbaren Pulvers vermischt werden, welches in Wasser dispergierbar ist, oder sie können mit einem festen vorgranulierten Trägerstoff zur Bildung eines granulatförmigen Produktes vermischt werden.

Wenn gewünscht, kann eine genannte Verbindung der allgemeinen Formel I in einem mit Wasser nicht mischbaren Lösungsmittel, wie beispielsweise einem alicyclischen Keton, gelöst werden, das zweckmässigerweise gelösten Emulgator enthält, so dass die Lösung bei Zugabe zu Wasser selbstemulgierend wirkt. Andernfalls kann der Wirkstoff mit einem Emulgator vermischt und das Gemisch dann mit Wasser auf die gewünschte Konzentration verdünnt werden. Zudem kann der Wirkstoff in einem Lösungsmittel gelöst und danach mit einem Emulgator gemischt werden. Ein solches Gemisch kann ebenfalls mit Wasser auf die gewünschte Konzentration verdünnt werden. Auf diese Weise erhält man emulgierbare Konzentrate bzw. gebrauchsfertige Emulsionen.

Die Verwendung der genannten Mittel kann nach den im Pflanzenschutz bzw. in der Landwirtschaft üblichen Applikationsmethoden erfolgen. Das genannte Verfahren zur Bekämpfung von Fungi ist dadurch gekennzeichnet, dass man den zu schützenden Ort oder das zu schützende Gut z.B. Pflanzen, Pflanzenteile bzw. Samen, mit einer wirksamen Menge einer genannten Verbindung der allgemeinen Formel I bzw. eines genannten Mittels behandelt.

Die nachstehenden Beispiele illustrieren Aspekte der Erfindung.

### I. Herstellung der Wirkstoffe der Formel I:

### Beispiel 1 (Nicht Gegenstand der Erfindung)

Zu einer Suspension von 0,78 g Natriumhydrid (80 % in Öl) in 20 ml Dimethylformamid werden unter Argonbegasung bei 0°C 5 g 2-(2-Brommethyl-phenyl)-glyoxylsäuremethylester-O-methyloxim und 3,2 g β-Acetonaphthonoxim in 80 ml Dimethylformamid zugetropft, und das Reaktionsgemisch wird 4 Stunden bei 0°C nachgerührt. Nach beendeter Reaktion wird das Gemisch mit gesättigter Ammoniumchloridlösung hydrolysiert und dreimal mit Diäthyläther extrahiert. Die vereinigten organischen Phasen werden über wasserfreiem Natriumsulfat getrocknet, und das Lösungsmittel wird abdestilliert. Das zurückbleibende Öl wird an Kieselgel unter Verwendung von Diäthyläther/n-Hexan (1:1) als Laufmittel chromatographisch gereinigt, und das Produkt aus MethylenchIorid/Diäthyläther/n-Hexan kristallisiert.

Auf diese Weise erhält man das 2-[α-{[(1-[β-Naphthyl]-äthyl)imino]oxy}-o-toIyl]-glyoxylsäure-methylester-O-methyloxim als weisse Kristalle, Smp. 97-98°C. (MS: 390(4); 116)

Analog dem in Beispiel 1 beschriebenen Verfahren erhält man aus dem entsprechenden o-substituierten Benzylbromid der Formel III (U = Br) und dem entsprechenden Oxim der Formel II die in der nachfolgenden Tabelle aufgeführten Verbindungen der Formel I.

Sie sind zum Teil durch ausgewählte Werte ihres Massenspektrums charakterisiert: Der erste Wert entspricht der höchsten Absorption. Der zweite Wert entspricht dem Basis-peak. In Klammern erscheint die Intensität des höchsten peaks in Prozent, bezogen auf den Basis-peak (= 100 %).

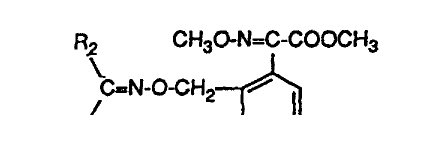

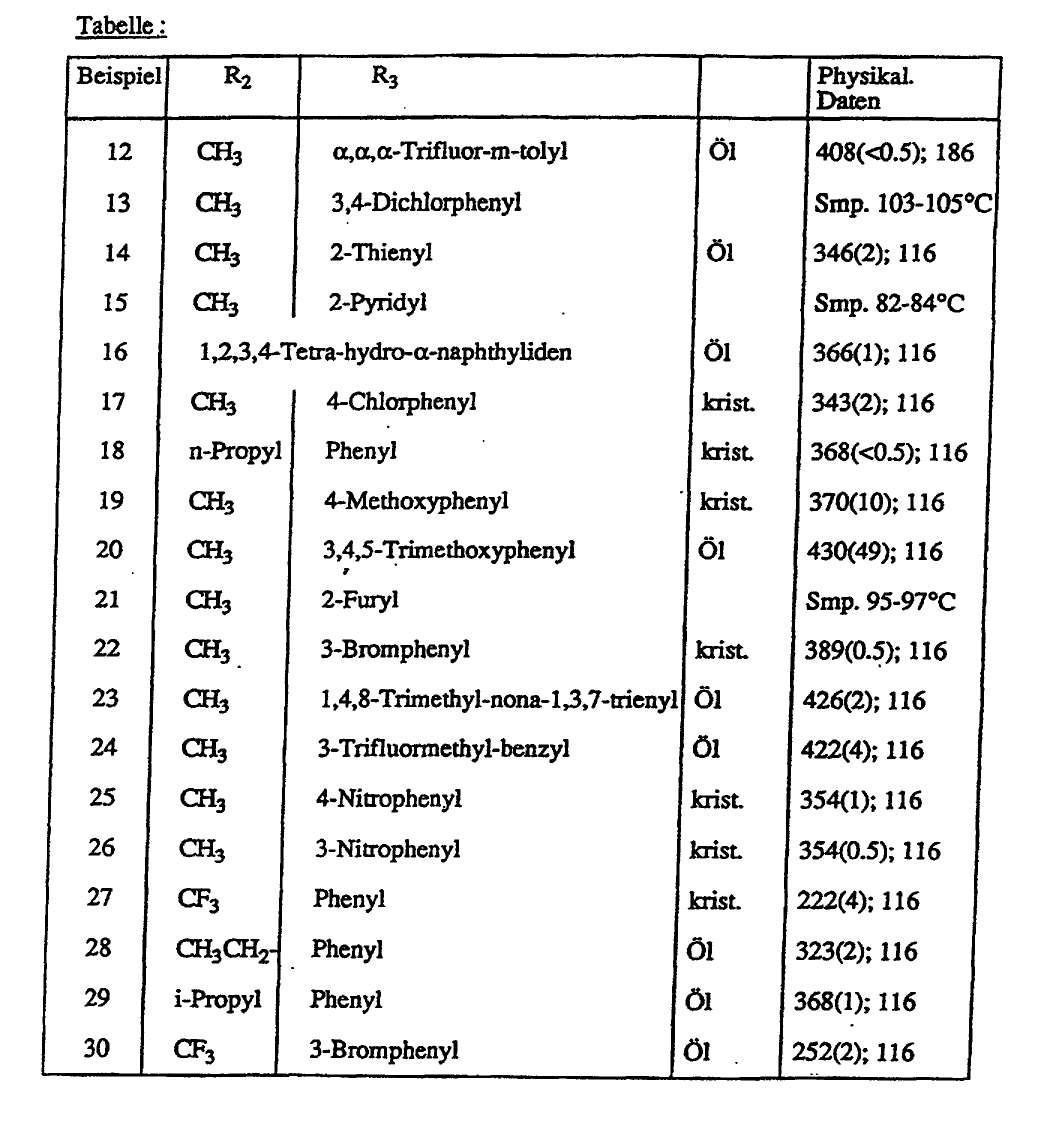

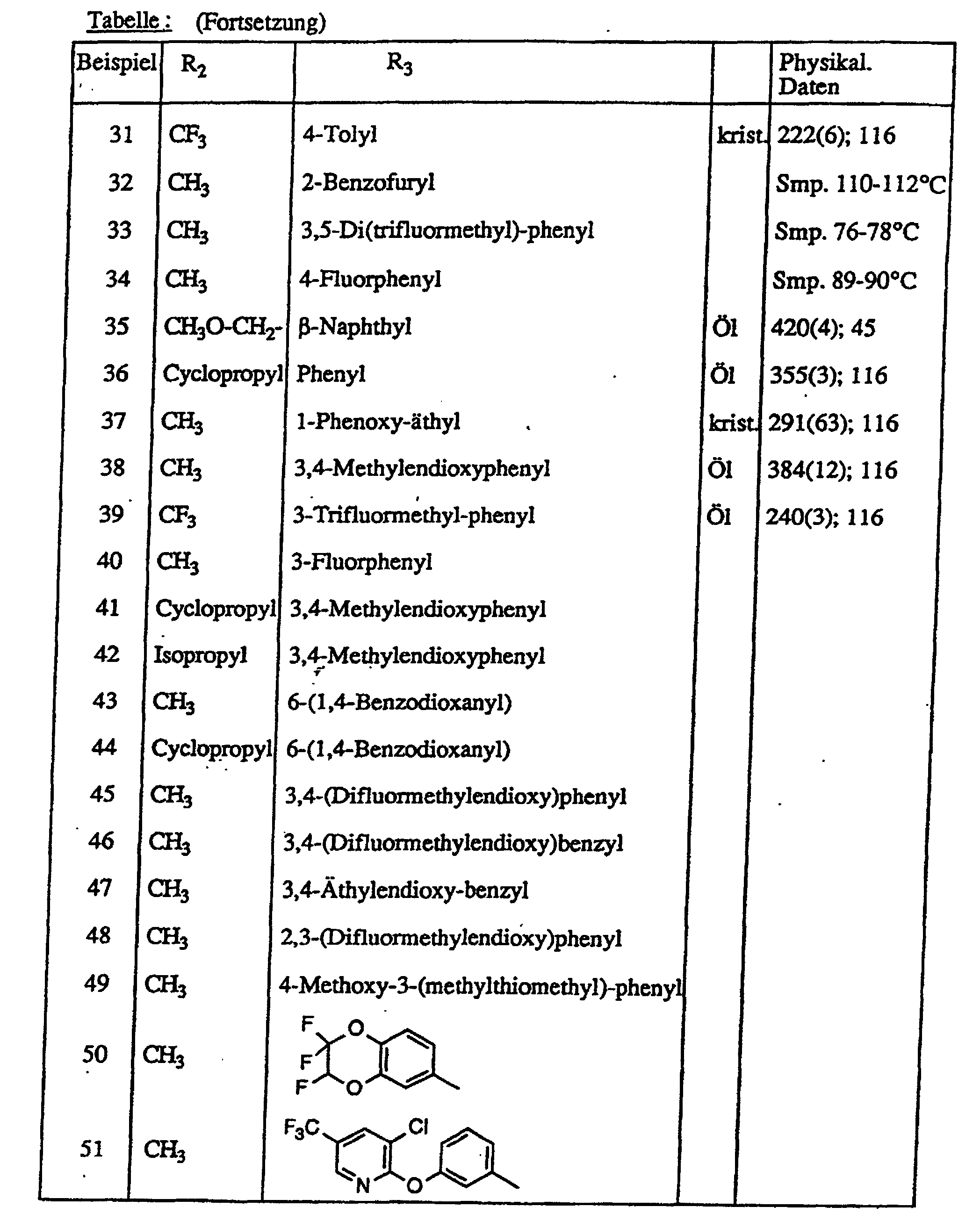

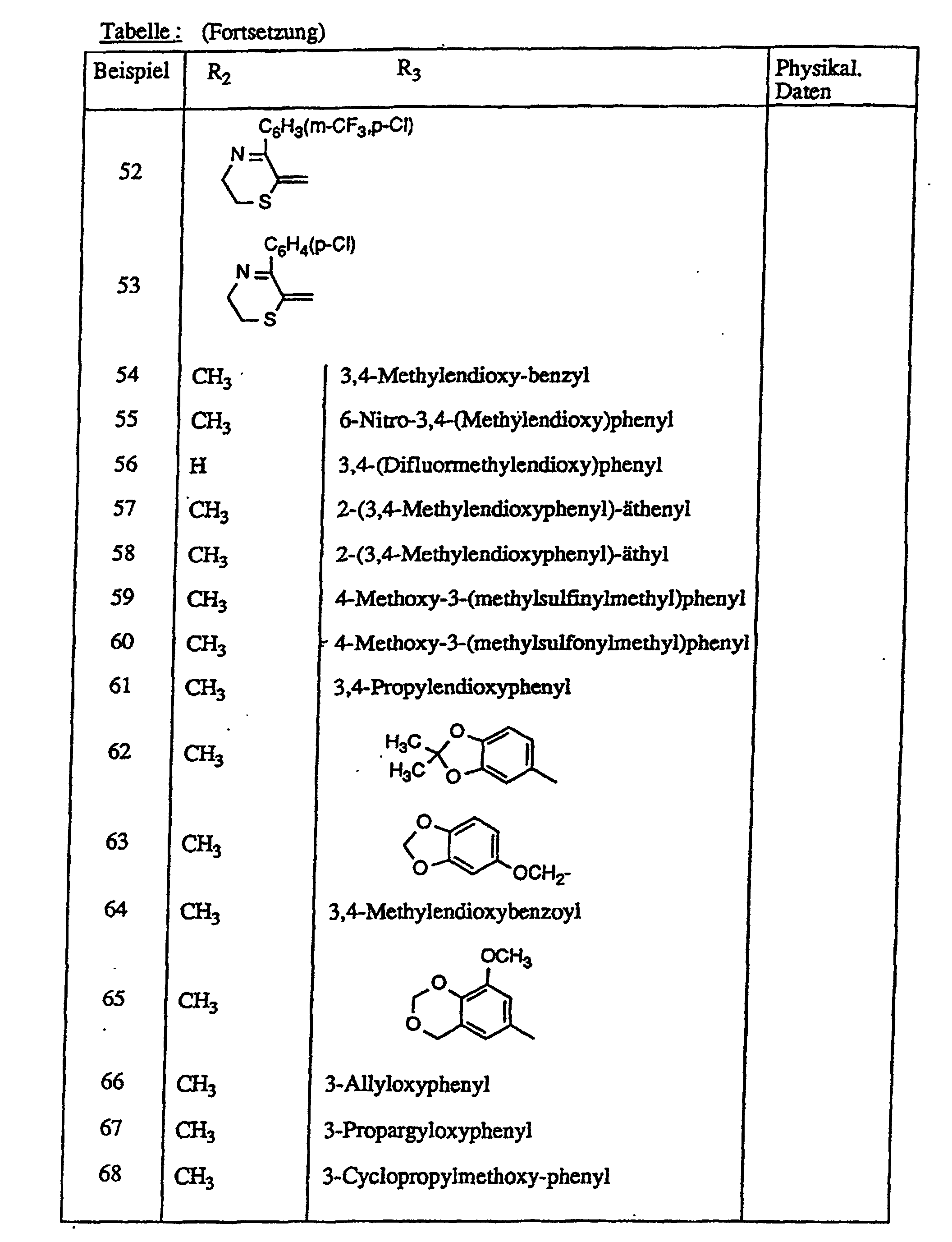

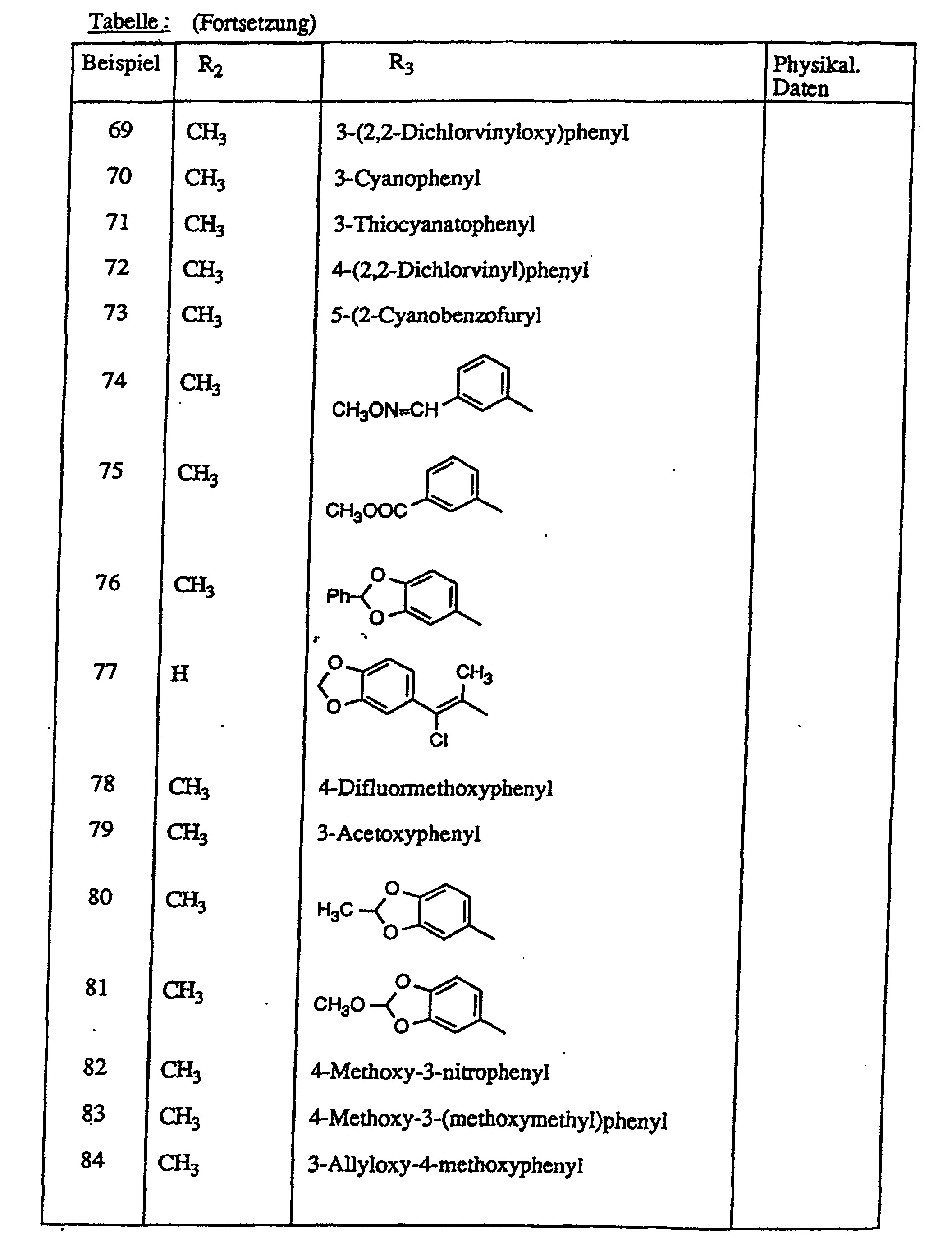

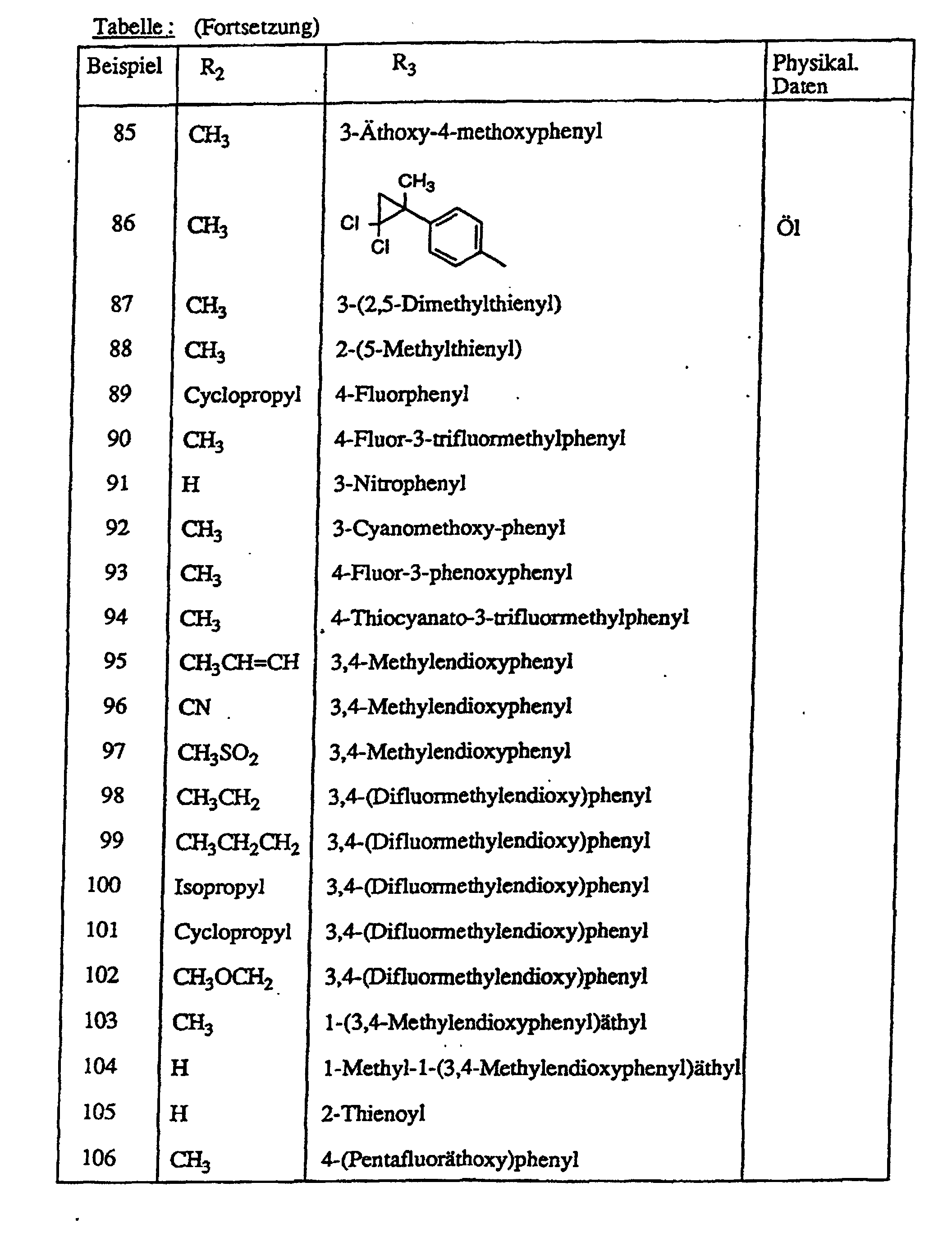

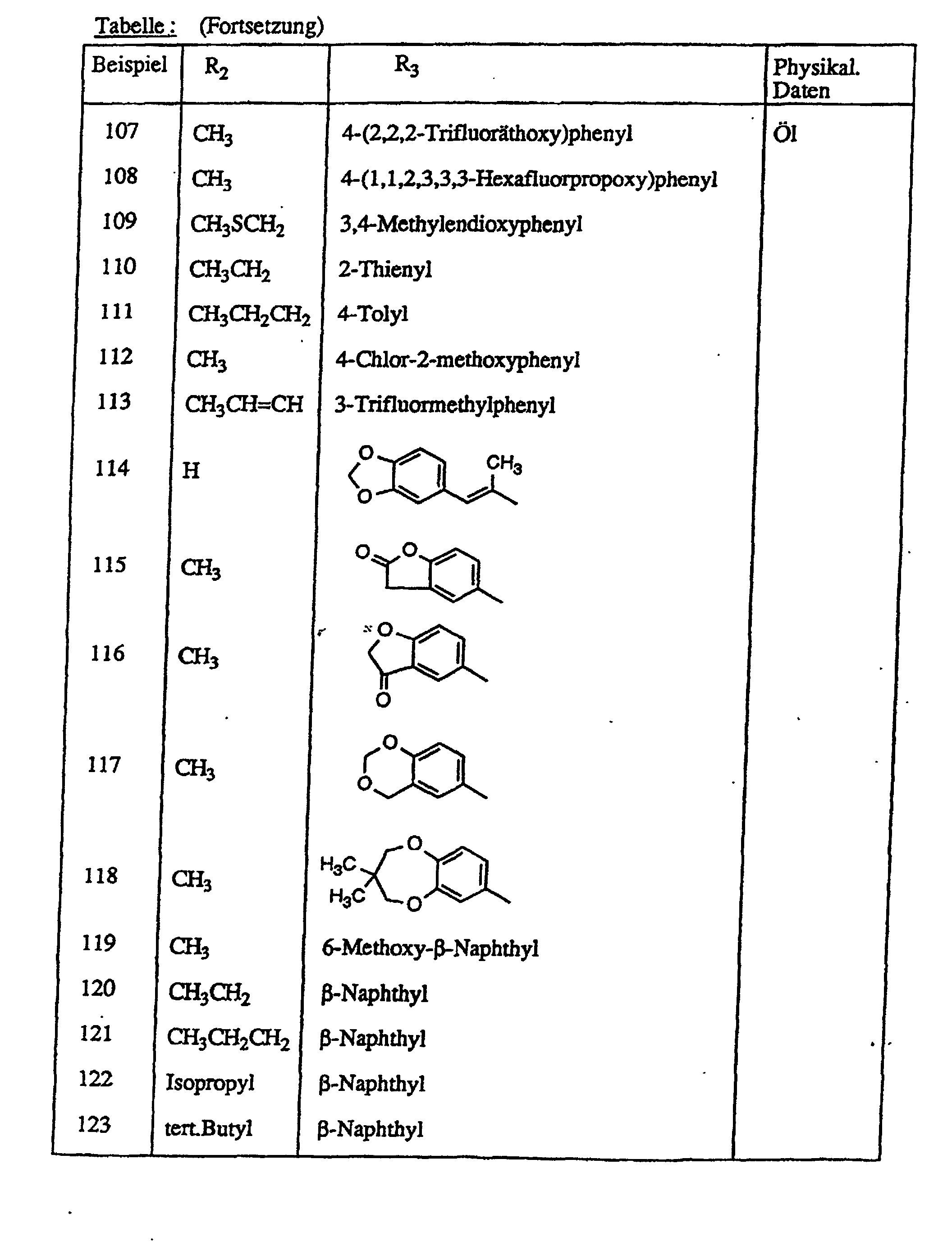

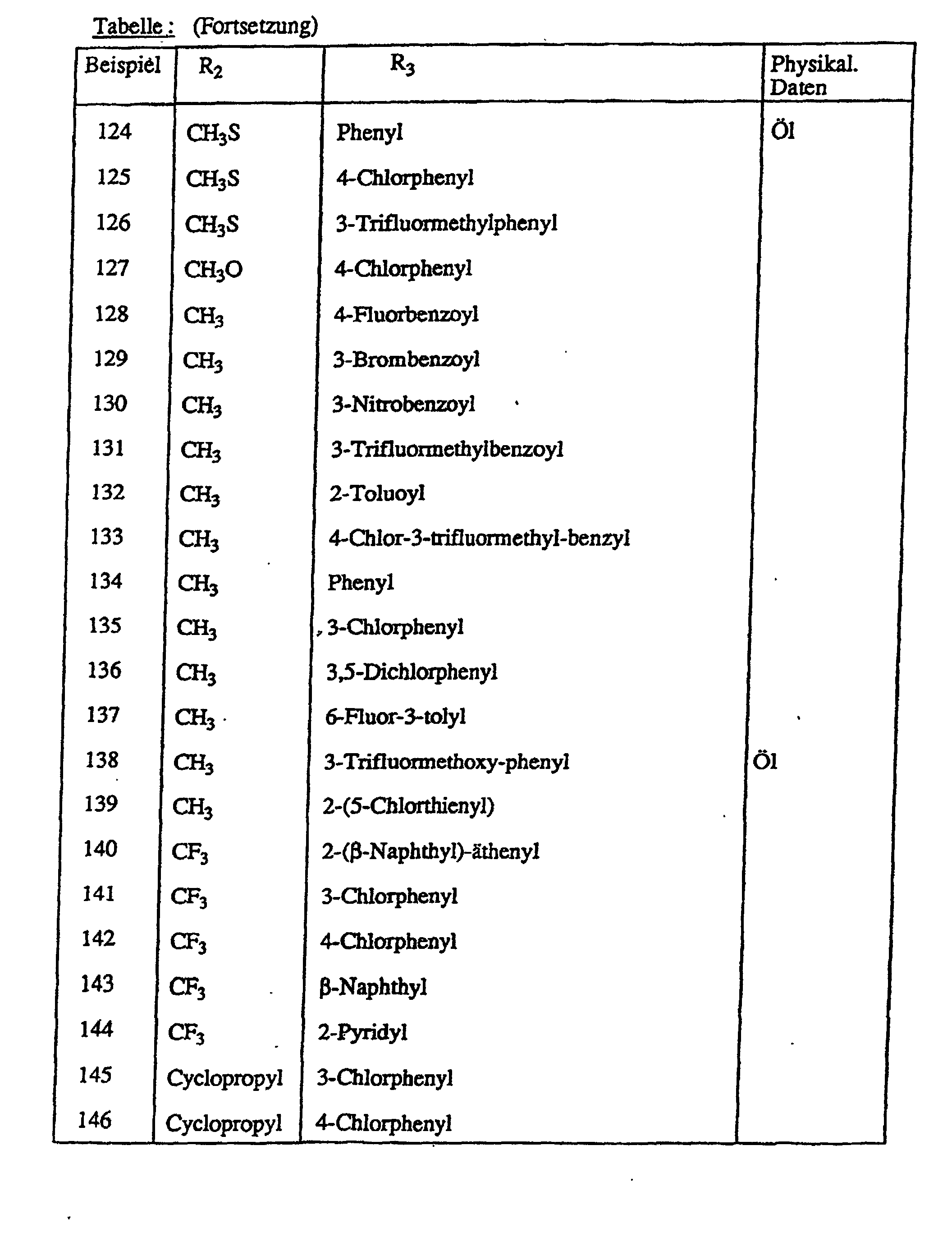

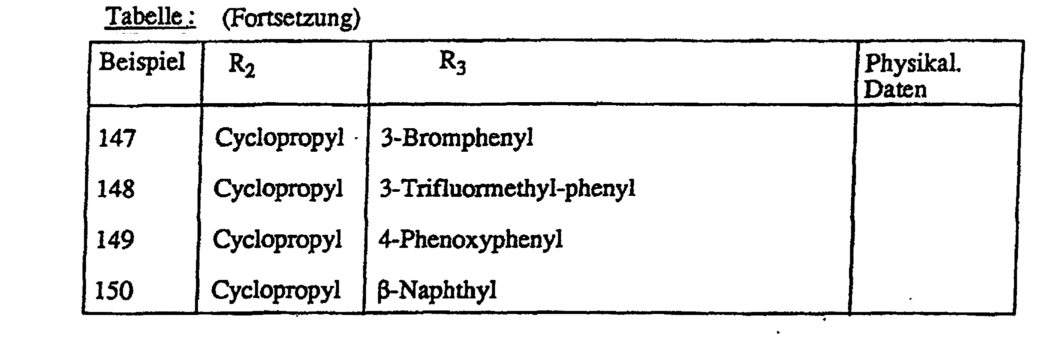

### Formulierungsbeispiele

### F1:

Ein emulgierbares Konzentrat hat z.B. folgende Zusammensetzung:

| | g/Liter |
|---|---|
| Wirkstoff der Tabelle | 100 |
| Nonylphenol-(10)äthoxylat (nicht ionischer Emulgator) | 50 |
| Calcium-dodecylbenzolsulfonat (anionischer Emulgator) | 25 |
| N-Methyl-2-pyrrolidon (Lösungsvermittler) | 200 |
| Gemisch von Alkylbenzolen (Lösungsmittel) | ad 1 Liter |

Der Wirkstoff und die Emulgatoren werden im Lösungsmittel und im Lösungsvermittler gelöst. Durch Emulgieren dieses Konzentrates in Wasser kann eine gebrauchsfertige Spritzbrühe beliebiger Verdünnung hergestellt werden.

### F2:

Ein Spritzpulver hat z.B. folgende Zusammensetzung:

| | Gewichtsprozent |
|---|---|
| Wirkstoff der Tabelle | 25,0 |
| Kieselsäure (hydrarisiert; Trägerstoff) | 20,0 |
| Natrium-IauryIsulfat (Netzmittel) | 2,0 |
| Natrium-lignosulfonat (Dispergiermittel) | 4,0 |
| Kaolin (Trägerstoff) | 49,0 |

Die Komponenten werden miteinander vermischt und in einer geeigneten Mühle feingemahlen. Durch Dispergieren des Gemisches in Wasser ergibt sich eine Suspension, die sich als gebrauchsfertige Spritzbrühe eignet.

### Biologische Beispiele:

### Beispiel B1: Puccinia coronata (kurative Wirkung)

30-40 Haferkeimlinge der Sorte "Selma" (verteilt auf 2 Töpfe mit 7 cm ⌀) werden durch Besprühen mit einer wässerigen Sporensuspension (ca. 150'000 Uredosporen/ml) mit *Puccinia coronata* infiziert. Anschliessend inkubiert man die Testpflanzen während 24 Std. bei 20-24°C und Taupunktbedingungen. Danach werden die Haferkeimlinge mit einer aus einem Spritzpulver des Wirkstoffes hergestellten Spritzbrühe (mit 160 ppm Aktivsubstanz) allseitig gründlich besprüht. Die weitere Kultivierung erfolgt in einer Klimakabine bei 19°C und einer Photoperiode von 14 Std. Die Versuchsauswertung erfolgt 9-10 Tage nach der Infektion durch Ermittlung der von *Puccinia coronata* befallenen Blattfläche in Prozent gegenüber der infizierten, nicht behandelten Kontrolle.

Über 75 % Wirkung bei 160 ppm zeigen z.B. folgende Verbindungen:
12, 17, 78, 90, 107, 128, 131, 133, 138, 148.

Unbehandelte aber infizierte Kontrollpflanzen zeigten einen Puccinia-Befall von 100 %.

### Beispiel B2: Wirkung gegen Cercospora arachidicola auf Erdnusspflanzen (kurative Wirkung)

Zwei Erdnusspflanzen der Sorte "Tamnut" werden im 4-Blattstadium mit einer Konidiensuspension von **Cercospora arachidicola** (ca. 200'000 Konidien/ml) besprüht und anschliessend bei 25-26°C und Taupunktbedingungen inkubiert. Nach zwei Tagen besprüht man die Planzen allseitig gründlich mit einer aus einem Spnitzpulver des Wirkstoffes hergestellten Spritzbrühe (mit 160 ppm Aktivsubstanz). Die behandelten Pflanzen werden anschliessend in einer Klimakabine inkubiert bei folgenden Bedingungen: 25-27°C und 80 % Luftfeuchtigkeit bei Tag, 20°C und Taupunktbedingungen während der Nacht; die Photoperiode beträgt jeweils 16 Stunden. 12 Tage nach der Behandlung erfolgt die Versuchsauswertung durch Ermittlung der von Cercospora arachidicola befallenen Blattfläche in Prozent gegenüber der infizierten Kontrolle.

Im Vergleich zu unbehandelten, aber infizierten Kontrollpflanzen (Anzahl und Grösse der Flecken = 100 %), zeigen Erdnusspflanzen, die mit Wirkstoffen aus den Tabellen behandelt wurden, einen stark reduzierten Cercospora-Befall.

Über 75 % Wirkung bei 160 ppm zeigen z.B. folgende Verbindungen:
12, 13, 14, 17, 19, 22, 24, 25, 26, 30, 32, 33, 34, 36, 38, 40, 41, 44, 49, 52, 66, 68, 71, 83, 90, 101, 129, 130, 133, 138, 145, 148.

### Beispiel B3: Erysiphe graminis (protektive Wirkung)

30-40 Weizenkeimlinge der Sorte "Lita" (verteilt auf 2 Töpfe mit 7 cm ⌀) werden im 1-Blattstadium mit einer aus einem Spritzpulver des Wirkstoffes hergestellten Spritzbrühe (mit 160 ppm Aktivsubstanz) gründlich besprüht und anschliessend im Gewächshaus weiterkultiviert. Einen Tag nach der Behandlung bestäubt man die Pflanzen mit Konidien von Erysiphe graminis. Die Versuchsauswertung erfolgt 7 Tage nach der Infektion durch Ermittlung der von Erysiphe graminis überwachsenen Blattoberfläche in Prozent gegenüber der infizierten Kontrolle.

Über 75 % Wirkung bei 160 ppm zeigen z.B. folgende Verbindungen:
1, 13, 15, 16, 18, 23, 24, 25, 27, 29, 31, 33, 34, 37, 39, 40, 52, 53, 70, 86, 89, 105, 119, 120, 126, 127, 128, 135, 137, 141, 149.

Unbehandelte, aber infizierte Kontrollpflanzen zeigen einen Erysiphae-Befall von 100 %.

### Beispiel B4: Venturia inaequalis (kurative Wirkung)

Zwei Apfelsämlinge der Sorte "Golden Delicious" werden mit einer Konidiensuspension von Venturia inaequalis besprüht und anschliessend bei 18°C und Taupunktbedingungen inkubiert. Nach 24 Stunden besprüht man die Pflanzen allseitig gründlich mit einer aus einem Spritzpulver des Wirkstoffes hergestellten Spritzbrühe (mit 50 ppm Aktivsubstanz). Die behandelten Apfelsämlinge werden anschliessend im Gewächshaus weiterkultiviert. 9-10 Tage nach der Behandlung erfolgt die Versuchsauswertung durch Ermittlung der von Venturia inaequalis überwachsenen Blattoberfläche in Prozent gegenüber der infizierten Kontrolle.

Über 75 % Wirkung bei 50 ppm zeigen z.B. folgende Verbindungen:
12, 13, 14, 15, 17, 19, 20, 22, 24, 28, 29, 30, 31, 32, 33, 34, 36, 38, 44, 49, 54, 61, 64, 66, 78, 82, 83, 85, 105, 106, 117, 124, 131, 134, 135, 139, 142, 147.

### Beispiel B5: Alternaria brassicae (protektive Wirkung)

4 Kohlsämlinge, Sorte "Vorbote", verteilt auf 2 Töpfe, werden im 6-Blattstadium mit einer aus einem Spritzpulver des Wirkstoffes hergestellten Spritzbrühe (mit 50 ppm Aktivsubstanz gründlich besprüht und anschliessend in einer Klimakabine bei 19°C und 16 Std. Beleuchtung pro Tag weiterkultiviert. Zwei Tage nach der Behandlung erfolgt die Infektion der Pflanzen durch Besprühen mit einer wässerigen Konidiensuspension (ca. 30'000 Konidien/ml). Danach werden die Kohlpflanzen bei 24-26°C, Taupunktbedingungen und einer Fotoperiode von 16 Std. inkubiert. Die Versuchsauswertung erfolgt 2-5 Tage nach der Infektion durch Ermittlung der von Alternaria brassicae befallenen Blattfläche in Prozent gegenüber der infizierten, nicht behandelten Kontrolle.

Über 75 % Wirkung bei 50 ppm zeigen z.B. folgende Verbindungen:
1, 12, 13, 14, 17, 20, 22, 23, 24, 25, 26, 28, 29, 30, 31, 32, 33, 34, 36, 38, 54, 55, 67, 92, 105, 124, 130, 136, 138, 143, 150.

### Beispiel B6: Wirkung gegen Phytophthora infestans auf Tomaten

### a) Kurative Wirkung

Tomatenpflanzen der Sorte "Roter Gnom" werden nach dreiwöchiger Anzucht mit einer Zoosporensuspension des Pilzes besprüht und in einer Kabine bei 18 bis 20° und gesättigter Luftfeuchtigkeit inkubiert. Unterbruch der Befeuchtung nach 24 Stunden. Nach dem Abtrocknen der Pflanzen werden diese mit einer Brühe besprüht, die die als Spritzpulver formulierte Wirksubstanz in einer Konzentration von 200 ppm enthält. Nach dem Antrocknen des Spritzbelages werden die Pflanzen wieder in der Feuchtkabine während 4 Tagen aufgestellt. Anzahl und Grösse der nach dieser Zeit aufgetretenen typischen Blattflecken sind der Bewertungsmassstab für die Wirksamkeit der geprüften Substanzen.

### b) Präventiv-Systemische Wirkung

Die als Spritzpulver formulierte Wirksubstanz wird in einer Konzentration von 60 ppm (bezogen auf das Bodenvolumen) auf die Bodenoberfläche von drei Wochen alten eingetopften Tomatenpflanzen der Sorte "Roter Gnom" gegeben. Nach dreitägiger Wartezeit wird die Blattunterseite der Pflanzen mit einer Zoosporensuspension von Phytophthora infestans besprüht. Sie werden dann 5 Tage in einer Sprühkabine bei 18 bis 20°C und gesättigter Luftfeuchtigkeit gehalten. Nach dieser Zeit bilden sich typische Blattflecken, deren Anzahl und Grösse zur Bewertung der Wirksamkeit der geprüften Substanzen dienen.

Verbindungen aus der Tabelle erzielen eine Hemmung des Krankheitsbefalls auf unter 20 %.

### Beispiel B7: Plasmopara viticola (protektive Wirkung)

2 Rebstecklinge der Sorte Riesling x Sylvaner werden jeweils im 4-5 Blattstadium mit einer aus einem Spritzpulver des Wirkstoffes hergestellten Spritzbrühe (mit 160 ppm Aktivsubstanz) allseitig gründlich besprüht und anschliessend in einer Klimakabine bei 17°C, 70-80 % rel. Luftfeuchtigkeit und einer Photoperiode von 16 Stunden weiterkultiviert. Nach 6 Tagen erfolgt die Infektion der Versuchspflanzen durch Besprühen der Blattunterseiten mit in destilliertem Wasser suspendierten Zoosporangien (ca. 300'000 Sporangien/ml) von Plasmopara viticola. Danach werden die Rebpflanzen wie folgt inkubiert: 1 Tag bei 22°C und Taupunktbedingungen im Dunkeln und anschliessend 4 Tage im Gewächshaus. Um die Fruktifikation von Plasmopara viticola zu induzieren, werden die Reben am 5. Tag nach der Infektion in eine Klimakabine mit Taupunktbedingungen und 22°C überführt.

Die Versuchsauswertung erfolgt jeweils am 6. Tag nach der Infektion durch Ermittlung der durch Plasmopara viticola befallenen Blattfläche in % gegenüber der infizierten, nicht behandelten Kontrolle.

Über 75 % Wirkung bei 160 ppm zeigen z.B. folgende Verbindungen: 1, 12, 17, 22, 24, 28, 29, 30, 31, 32, 36, 38, 41, 43, 45, 53, 61, 62, 82, 102, 107, 120, 125, 129, 135, 138, 147, 150.

## Patentansprüche

1. Verbindungen der allgemeinen Formel IIIc 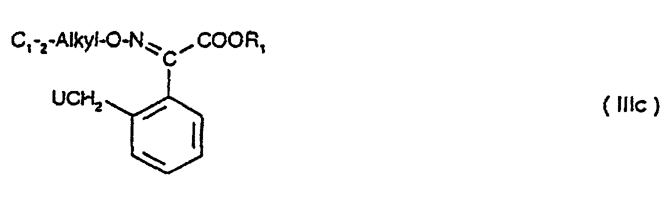 worin
R₁ für C₁₋₄-Alkyl und
U für Chlor, Brom, Jod, Mesyloxy, Benzolsulfonyloxy oder Tosyloxy stehen mit der Massgabe, dass R₁ und C₁₋₂-AIkyl nicht gleichzeitig Methyl bedeuten wenn U für Brom steht.

## Claims

1. Compounds of the general formula IIIc 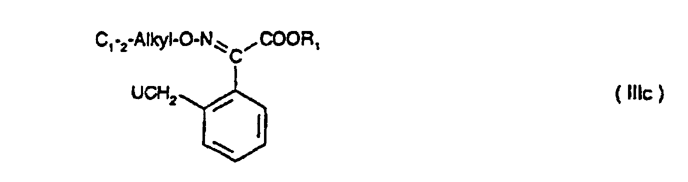 in which
R₁ represents C₁₋₄-alkyl and
U represents chlorine, bromine, iodine, mesyloxy, benzenesulphonyloxy or tosyloxy with the proviso that R₁ and C₁₋₂-alkyl are not simultaneously methyl if U represents bromine.

## Revendications

1. Composés de formule générale IIIc 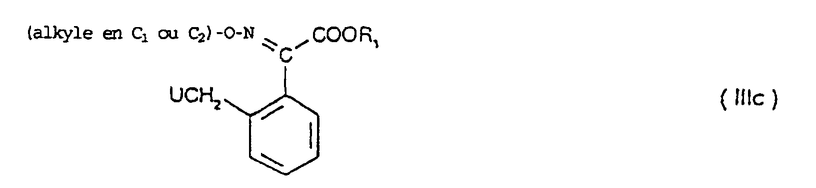 dans laquelle
R₁ représente un reste alkyle en C₁ à C₄ et
U représente le chlore, le brome, l'iode, un reste mésyloxy, benzènesulfonyloxy ou tosyloxy, sous réserve que R₁ et le reste alkyle en C₁ ou C₂ ne désignent pas en même temps un reste méthyle lorsque U représente le brome.
